(19) **Europäisches Patentamt / European Patent Office / Office européen des brevets**

(11) **EP 1 393 680 B1**

(12) **EUROPEAN PATENT SPECIFICATION**

(45) Date of publication and mention
of the grant of the patent:
**01.08.2007 Bulletin 2007/31**

(51) Int Cl.:
*A61B 6/00* (2006.01)  *A61B 8/08* (2006.01)

(21) Application number: **02019416.3**

(22) Date of filing: **30.08.2002**

(54) **Apparatus for bone mineral density measurement**

Vorrichtung zur Messung der Knochen-Mineraldichten

Dispositif destiné à mesurer la densité de minéraux osseux

(84) Designated Contracting States:
**DE DK FR**

(43) Date of publication of application:
**03.03.2004 Bulletin 2004/10**

(73) Proprietor: **L'ACN L'ACCESSORIO NUCLEARE SRL**
**20023 Cerro Maggiore (Milano) (IT)**

(72) Inventor: **Pepe, Leonardo**
**20017 Rho (Milano) (IT)**

(74) Representative: **Petruzziello, Aldo**
**Racheli & C. S.p.A**
**Viale San Michele del Carso, 4**
**20144 Milano (IT)**

(56) References cited:
**EP-A- 0 480 554**   **EP-A- 0 570 936**
**US-A- 4 829 549**   **US-A- 5 218 963**
**US-A- 6 086 538**

Printed by Jouve, 75001 PARIS (FR)

EP 1 393 680 B1

## Description

The present invention refers to an apparatus and a method for computerised bone mineral density measurement (BMD).

Bone mineral density measurement is based on measurement of the mineral content in the bones of a patient with the aim of evaluating bone density for possible diagnosis of osteoporosis.

Two bone mineral density measurement systems are currently known to the art: the first based on the use of X-rays and the second based on the use of ultrasound.

The X-ray bone mineral measurement technique is based on the law of absorption of X-rays by the calcium contained in bones and is used on bones such as those of the spine, the femur and the forearm. The examination is conducted by scanning a region of interest (R.O.I.) by means of a suitably collimated X-ray beam received by a detector.

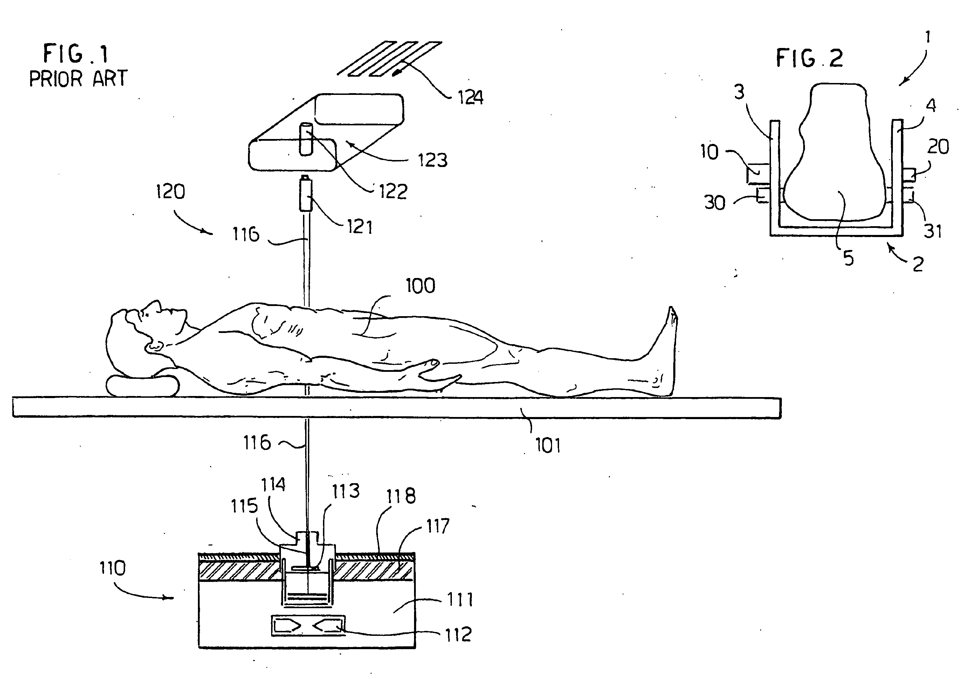

An apparatus for X-ray bone mineral density measurement according to the prior art, wherein the R.O.I. is represented by the lumbar spine, is described hereunder with reference to Figure 1.

The X-ray apparatus comprises an X-ray source assembly 110 and an X-ray receiving or detecting assembly 120 disposed respectively below and above a horizontal bed 101 intended to accommodate the patient 100 in a supine position.

The emission assembly 110 comprises a tank filled with cooling oil 111 contained in a monoblock 117 covered with a lead screen 118.

An X-ray tube or radiation source 112 which emits an X-ray beam (at a voltage of about 86 kV) is immersed in the cooling oil. Above the X-ray tube 112, in the monobloc 117, a samarium filter 113 is provided which filters the beam emitted by the X-ray tube 112 and converts it into a dual energy-band beam, the lowest of which refers to the characteristic line of the samarium set at 37 keV.

Positioned above the samarium filter 113 is a collimator 114 which has a collimation hole 115 that protrudes from the lead screen 118. The collimation hole 115 is able to collimate the dual energy-band X-ray beam leaving the samarium filter 113.

Consequently a dual energy-band collimated beam 116, commonly known as a pencil beam, leaves the collimation hole 115.

The detector assembly 120 comprises a scintillator or crystal detector with a photomultiplier 121 and a pre-amplifier 122 mounted on a mobile arm 123 able to permit movement on two axes according to a particular path shown in 124 to allow multi-line scanning for reconstruction of the image.

The emission assembly 110 also, during scanning of the R.O.I., must move synchronus with the detector assembly 120, so that the scintillator 121 remains coaxial with respect to the collimator hole 115 of the emission assembly 110.

The scintillator 121 captures the photons that pass through the R.O.I. and transforms them into electrical pulses having a height proportional to the emission energy. The pre-amplifier 122 amplifies the signal detected by the scintillator and sends it to a processing unit for processing of the data received.

The bone mineral density (BMD), which is the main parameter for evaluation of the calcium in the bone, is calculated from the data received. BMD is calculated according to the following formula:

$$ BMD = \frac{RST * \log\left(\dfrac{Ih}{Iho}\right) - \log\dfrac{Il}{Ilo}}{Al - RST * Ah} $$

wherein:

BMD = bone mineral density expressed in g/cm$^2$
RST = soft tissue subtraction coefficient
Ih = Intensity of the high-energy radioactive beam in the ROI
Il = Intensity of the low-energy radioactive beam in the ROI
Iho= Intensity of the high-energy radioactive beam in air
Ilo = Intensity of the low-energy radioactive beam in air

The BMD result obtained is compared with normal values acquired on a "universal" population and the medical report is written on the BMD analysis. In fact, low BMD values indicate a tendency for the patient to develop osteoporosis, and accordingly suggest any indications for treatment.

The ultrasound bone mineral density measurement system will now be described.

The ultrasound technique is based on measurement of the speed of transmission and attenuation of sound within the bone, and is used above all on the heel bone.

The ultrasound signal is transmitted by a transmitting transducer and received on the opposite side of the bone by a receiving transducer. The following parameters are calculated from the analysis of the signal received:

- Broadband ultrasonic attenuation (BUA);
- Speed of sound (SOS)

With respect to X-ray BMD measurement, which gives a direct indication of the calcium content of the bone, ultrasound BMD measurement gives an indication of the state of porosity and elasticity of the bone. Ultrasound BMD is, therefore, an indirect measurement of bone density and of its calcium content and can be considered, to all effects, as a complementary measurement to X-ray BMD.

There currently exist on the market X-ray BMD systems and ultrasound BMD systems. Consequently a patient at risk for osteoporosis, depending upon his needs and the equipment available at the medical centre, is directed towards an X-ray BMD machine or an ultrasound BMD machine.

Both X-ray BMD machines and ultrasound BMD machines have some drawbacks. In fact only one measurement is made on each machine, X-ray or ultrasound, which cannot be compared with other measurements, to the detriment of its accuracy and reproducibility.

EP 0 570 936 discloses a bone assessment apparatus comprising means of X-ray for measuring the mineral density of bone (BMD) and means of ultrasonic waves for measuring the propagation speed of the sound in the bone.

US 6,086,538 discloses a method and apparatus for evaluation of bone condition which provides means of ultrasonic waves for measurements of broadband ultrasonic attenuation (BUA) and the speed of sound transmission in the bone (SOS).

The object of the present invention is to provide an apparatus and a method for bone mineral density measurement that overcome the drawbacks of the prior art and are able to provide a good level of reliability, accuracy and reproducibility of the measurement.

Another object of the present invention is to provide an apparatus and a method for bone mineral density measurement that are able to provide a plurality of measurements that can be compared with one another.

Another object of the present invention is to provide such an apparatus for bone mineral density measurement that is efficient, versatile and occupies little space.

These objects have been achieved in accordance with the invention with the method according to independent claim 1 and with the apparatus according to independent claim 4.

Advantageous embodiments of the invention are apparent from the dependent claims.

The apparatus for bone mineral density measurement according to the invention comprises an X-ray source coupled to an X-ray receiver and an ultrasound transmitter coupled to an ultrasound receiver. In this manner bone mineral density analyses can be made both with the X-ray measurement method and with the ultrasound measurement method.

With the X-ray measurement method a point of minimum bone thickness can be identified in the region of interest analysed. Then, with the ultrasound technique bone mineral density measurement is carried out in the point of minimum bone thickness previously identified. Lastly, the measurements made with the X-ray technique are compared with those made with the ultrasound technique so as to obtain good accuracy and reliability of the measurement method.

Further characteristics of the invention are made clearer by the detailed description that follows, referring to a purely exemplary and therefore non-limiting embodiment thereof, illustrated in the appended drawings, in which:

Figure 1 is a diagrammatic view illustrating an apparatus for X-ray bone mineral density measurement according to the prior art;

Figure 2 is a diagrammatic view, illustrating an apparatus for bone mineral density measurement according to the invention;

Figure 3 is a front view, partially in section, diagrammatically illustrating the apparatus for bone mineral density measurement according to the invention;

Figure 4 is side elevational view, partially in section, of the apparatus for bone mineral density measurement of Figure 3.

Figure 5 is a sectional view along the sectional plane V-V of Figure 3;

Figure 6 is a sectional view along the sectional plane VI-VI of Figure 3;

Figure 7 is a block diagram illustrating some components of the apparatus for bone mineral density measurement according to the invention.

An apparatus for computerised bone mineral density (BMD) measurement according to the invention, designated as a whole with reference numeral 1, is described with the aid of the figures.

Specific reference will be made hereunder to a BMD apparatus particularly suited to be used for use in measurement of the heel bone, it being understood that the invention extends to such an apparatus suitable to perform measurements in other parts of the patient's body also.

Figure 2 diagrammatically illustrates an apparatus 1 for BMD according to the invention. The apparatus 1 comprises a frame 2 that is substantially U-shaped in cross section. The frame 2 comprises a first supporting shoulder 3 opposite to a second supporting shoulder 4.

The first shoulder 3 supports an X-ray source assembly 10 and an ultrasound transmitting transducer 30 and the second shoulder 4 supports an X-ray receiving or detecting assembly 20 and an ultrasound receiving or detecting transducer 31.

The two shoulders 3, 4 are disposed at such a distance as to allow insertion therebetween of a heel 5, so as to be able to perform a BMD measurement on the heel bone.

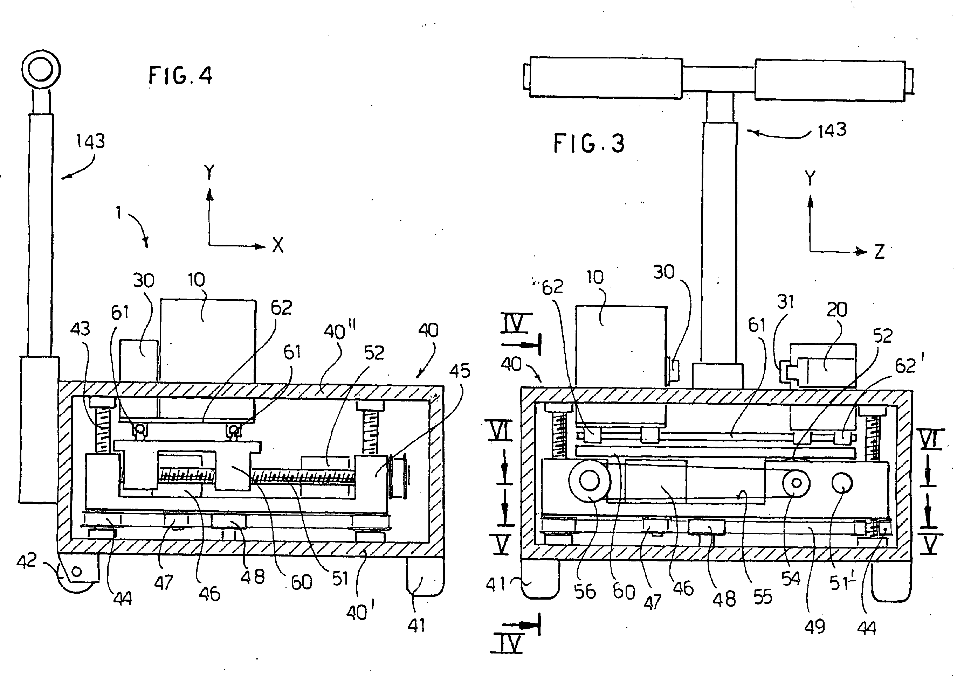

The apparatus for BMD measurement according to the invention will now be described in greater detail with reference to Figures 3-6.

The apparatus 1 comprises a substantially parallelepiped-shaped box 40. The box 40 is supported at the front by two feet 41 for standing and at the rear by two wheels 42 for pulling. A substantially T-shaped pulling handle 143 is fixed in the rear wall of the box 40.

Between the bottom wall 40' and the top wall 40" of the box 40 there are mounted four threaded uprights or screws 43 (Figure 4), disposed at the four corners of the bottom wall 40' and at right angles thereto. Respective lead or nut screws 44, which support a vertical movement plate 45 which has four holes wherein the four screws 43 pass, engage on the four screws 43. In this manner the vertical movement plate 45 can translate vertically in the direction of the axis Y, supported by the four nut screws 44.

A motor 46 having a shaft at right angles to the plate 45 with a pinion 47 disposed beneath the plate 45 is mounted on the vertical movement plate 45 for vertical movement. A idle pulley 48 disposed in a substantially central position is rotatably mounted beneath the vertical movement plate 45.

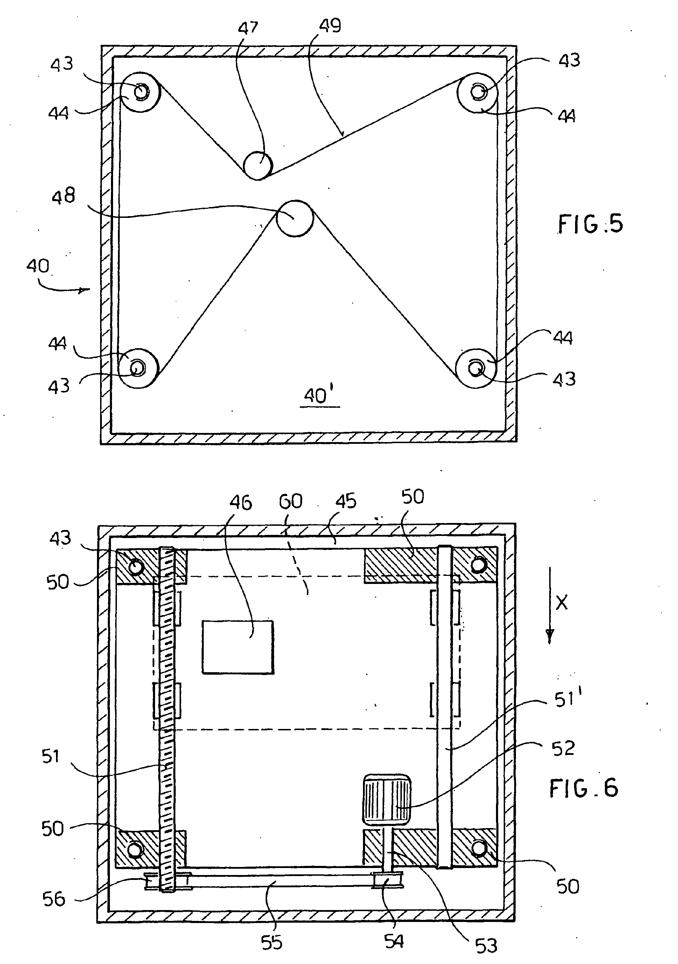

A cog belt 49 is driven by the pinion 47 and directed through the four nut screws 44 and the idle pulley 48, following a substantially butterfly-shaped course (Figure 5). In this manner, the rotation of the pinion 47 drives the cog belt 49 which sets simultaneously in rotation the four nut screws 44 which screw/unscrew in the respective screws 43 raising or lowering the vertical movement plate 45.

As shown in Figure 6, the vertical movement plate 45 has four shoulders 50 at its four corners. Two screws 51, 51', disposed on a horizontal plane with its axis lying along the axis X, are rotatably mounted on the two pairs of shoulders 50.

A motor 52 for horizontal movement is mounted on the vertical movement plate 45. The shaft 53 of the motor 52 sets in rotation the two pinions 54, 54' which respectively drive two belts 55, 55'. The belts 55, 55' set in rotation two pulleys 56, 56' which set in rotation the respective screws 51, 51'.

On the horizontal screws 51, 51' there is mounted a horizontal movement plate 60, which has an internal thread that meshes with the external thread of the screws 51, 51', so that rotation of the screws 51, 51' causes a translation of the horizontal movement plate 60 along direction of the axis X.

Two guide rails 61 disposed parallel to each other and along a horizontal axis Z at right angles to the axis X are mounted on the horizontal movement plate 60. Two supporting plates 62 and 62' are slidably mounted on the guide rails 61. The first supporting plate 62 supports the X-ray source assembly 10 and the ultrasound transmitting transducer 30 and the second supporting plate 62' supports the X-ray receiving assembly 20 and the ultrasound receiving transducer 31.

In this manner, the supporting plates 62, 62' can be moved together or apart depending upon the R.O.I. whereon the BMD measurement must be made. Moreover, according to the scanning to be done on the R.O.I., in a plane X-Y, the motor 46 for vertical movement which causes the vertical movement plate 45 to translate vertically (along the axis Y) and the motor 52 for horizontal movement which causes the horizontal movement plate 60 to translate horizontally (along axis X) are started.

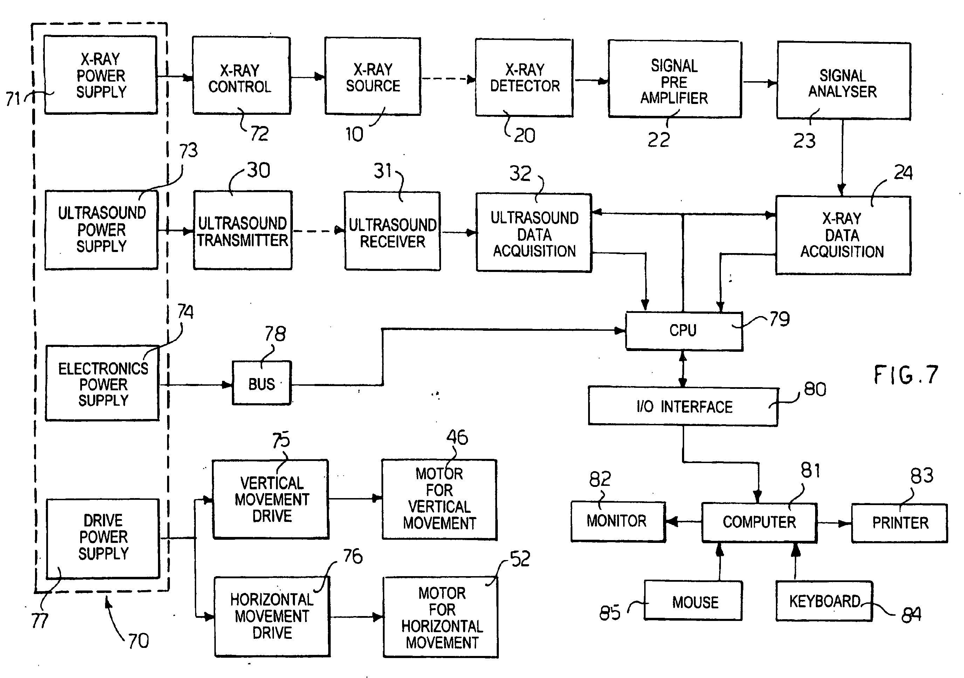

With reference to Figure 7, a block diagram illustrating the various components of the apparatus 1 for bone mineral density measurement according to the invention is shown. The apparatus 1 has a power supply system 70 that comprises a high-voltage power supply 71 which, through a control 72, is sent to an X-ray tube of the X-ray source assembly 10 which emits a dual-energy X-ray beam.

The X-ray beam is detected by the X-ray beam detector assembly 20, which according to the radiation detected sends an electric signal to a signal preamplifier 22 which in turn sends it to a signal analyser 23. The output of the signal analyser 23 is sent to a data acquisition system 24 able to acquire the data from the X-ray analysis.

The power supply system 70 further comprises an independent power supply 73 able to supply the ultrasound transmitting transducer 30 which has an ultrasound transmitter able to transmit ultrasound with a frequency of about 500 kHz. The transmitted ultrasound is received by the ultrasound receiving transducer 31 which, according to the ultrasound received, outputs an electrical output signal towards a data acquisition system 32 able to acquire the data from the ultrasound analysis.

The data acquisition systems 24 and 32 are controlled by a CPU 79 connected to an I/O interface 80 for connection to a computer 81. A monitor 82 to display the analysis and measurement data, a printer 83 for paper printout of the results of the measurements, a keyboard 84 and a mouse 85 to send commands to the apparatus 1 can be connected to the computer 81.

The power supply system 70 further comprises a low voltage power supply 77 to supply the electronics of the apparatus. The power supply 77 for the electronics is connected to a bus 78 which is responsible for powering the CPU 79 of the data acquisition systems.

Lastly, the power supply system 70 comprises a power supply 74 to power the drives 75 and 76 respectively of the motor 46 for vertical movement (along axis Y) and of the motor 52 for horizontal movement (along axis X).

Operation of the apparatus 1 for BMD measurement on the heel bone is described by way of example.

The patient's heel is positioned on the frame plate 40 of the apparatus 1, between the transmitter assembly comprising the X-ray source 10 and the ultrasound transmitter 30 and the receiving assembly comprising the X-ray receiver 20 and the ultrasound receiver 31. The transmitter assemblies 10, 30 and receiver assemblies 20, 31 are adjusted in position by making them translate on their supporting plates 62, 62' along the guide rails 61 disposed along the axis Z.

Then, the X-ray measurement is carried out. The X-ray source 10 and the X-ray receiver 20 are activated and the motors 46, 52 are started to scan an area of about 10 cm x 10 cm on the plane X-Y.

By means of the data acquired by the data acquisition system 24, the CPU 79 calculates the mean bone mineral density (BMD) value referred to the entire R.O.I.

Moreover, X-ray measurement identifies the point of minimum thickness of the heel bone that is taken as the reference point for the subsequent measurements.

In a second phase the ultrasound transmitter 30 and receiver 31 are activated to perform an ultrasound measurement in the point of minimum bone thickness previously identified. During the ultrasound measurement the data acquisition system 32 calculates the broadband ultrasound attenuation (BUA) and speed of sound (SOS) parameters.

Lastly, the measurements performed with the X-ray method are compared with those performed with the ultrasound method to establish the reliability of the result of the measurement.

Numerous variations and modifications of detail within the reach of a person skilled in the art can be made to the present embodiment of the invention without departing from the scope of the invention as set forth by the appended claims.

**Claims**

1. A method for bone mineral density measurement comprising the following steps:

   - scanning of a region of interest (R.O.I.) of the bone by means of an X-raybeam,
   - detection of the X-rays transmitted through the R.O.I. of the bone and calculation of the mean bone mineral density (BMD) in said R.O.I.,
   - identification of a point of minimum thickness of the bone in the R.O.I.,
   - scanning of said point of minimum thickness of the bone by means of ultrasound,
   - calculation of the ultrasound attenuation (BUA) and of the speed of sound (SOS) in said point of minimum thickness of the bone, and
   - comparison of the measurements performed with X-rays with the measurements performed with ultrasound at said point of minimum thickness of the bone.

2. A method according to claim 1, **characterised in that** in the region of interest (R.O.I.) a scanning area of about 10 cm x 10 cm is defined by X-rays.

3. A method according to claim 1 or 2, **characterised in that** it is applied to the bones of the heel.

4. An apparatus (1) for bone mineral density measurement comprising:

   - an X-ray source (10) for scanning of a region of interest (R.O.I.) of the bone by means of an X-ray beam,
   - an X-ray receiver/detector (20) for detection of the X-rays transmitted through the R.O.I. of the bone,
   - calculation means (79) for calculation of the mean bone mineral density (BMD) in said R.O.I and identification of a point of minimum thickness of the bone in the R.O.I.,
   - an ultrasound transmitter (30) for scanning of said point of minimum thickness of the bone by means of ultrasound,
   - an ultrasound receiver/detector (31) for detection of ultrasound waves through said point of minimum thickness of the bone,
   - calculation means (79) for calculation of the ultrasound attenuation (BUA) and of the speed of sound (SOS) in said point of minimum thickness of the bone, and
   - comparison means (79) for comparison of the measurements performed with X-rays with the measurements performed with ultrasound at said point of minimum thickness of the bone.

5. An apparatus according to claim 4, **characterised in that** said X-ray emitting assembly (10) is integral with said ultrasound transmitting assembly (30) and said X-ray receiving assembly (20) is integral with said ultrasound receiving assembly (31).

6. An apparatus according to claim 4 or 5, **characterised in that** said X-ray emitting assembly (10) and said ultrasound transmitting assembly (30) are mounted on a first supporting plate (62) and said X-ray receiving assembly (20) and said ultrasound receiving assembly (31) are mounted on a second supporting plate (62'), said first supporting plate (62) and said second supporting plate (62') being translatably mounted on guides (61) so as to be adjustable in position.

7. An apparatus according to any one of the claims 4 to 6, **characterised in that** said X-ray emitting (10) and said ultrasound transmitting assembly (30) and said X-ray receiving assembly (20) and said ultrasound receiving assembly (31) are driven, integrally with each other, along a defined scanning path on a plane (X-Y).

8. An apparatus according to claim 7, **characterised in that** said X-ray emitting (10) and ultrasound transmitting assembly (30) and said X-ray receiving (20) and said ultrasound receiving assembly (31) are mounted on a horizontal movement plate (60) horizontally translatable along an axis (X), said horizontal movement plate (60) being mounted on vertical movement plate (45) vertically translatable along an axis (Y).

9. An apparatus according to claim 8, **characterised in that** said horizontal movement plate (60) is mounted on screws (51) disposed horizontally and set in rotation by means of a belt (55) driven by a motor (52) for horizontal movement and said vertical movement plate (45) is mounted on nut screws (44) engaging in screws (43) disposed vertically, said nut screws (44) being driven by means of a belt (49) driven by a motor (46) for vertical movement.

**Patentansprüche**

1. Verfahren zum Messen der Knochen-Mineraldichte, das die folgenden Schritte umfasst:

   - Abtasten eines Bereichs von Interesse (R.O.I.) des Knochens mittels eines Röntgenstrahls,
   - Erfassen der durch den R.O.I. des Knochens übertragenen Röntgenstrahlen und Berechnen der mittleren Knochen-Mineraldichte (BMD) im R.O.I.,
   - Identifizieren eines Punktes der minimalen Dicke des Knochens im R.O.I.,
   - Abtasten des Punktes der minimalen Dicke des Knochens mittels Ultraschall,
   - Berechnen der Ultraschalldämpfung (BUA) und der Schallgeschwindigkeit (SOS) im Punkt der minimalen Dicke des Knochens und
   - Vergleichen der mit Röntgenstrahlen ausgeführten Messungen mit den mit Ultraschall ausgeführten Messungen am Punkt der minimalen Dicke des Knochens.

2. Verfahren nach Anspruch 1, **dadurch gekennzeichnet, dass** im Bereich von Interesse (R.O.I.) eine Abtastfläche von etwa 10 cm x 10 cm durch Röntgenstrahlen definiert wird.

3. Verfahren nach Anspruch 1 oder 2, **dadurch gekennzeichnet, dass** es auf die Knochen des Fersenbeins angewendet wird.

4. Vorrichtung (1) zum Messen der Knochen-Mineraldichte, die Folgendes umfasst:.

   - eine Röntgenstrahlenquelle (10) zum Abtasten eines Bereichs von Interesse (R.O.I.) des Knochens mittels eines Röntgenstrahls,
   - einen Röntgenstrahl-Empfänger/-Detektor (20) zum Erfassen der durch den R.O.I. des Knochens übertragenen Röntgenstrahlen,
   - Berechnungsmittel (79) zum Berechnen der mittleren Knochen-Mineraldichte (BMD) im R.O.I. und Identifizieren eines Punktes der minimalen Dicke des Knochens im R.O.I.,
   - einen Ultraschallstrahler (30) zum Abtasten des Punktes der minimalen Dicke des Knochens mittels Ultraschall,
   - einen Ultraschall-Empfänger/-Detektor (31) zum Erfassen von Ultraschallwellen durch den Punkt der minimalen Dicke des Knochens,
   - Berechnungsmittel (79) zum Berechnen der Ultraschalldämpfung (BUA) und der Schallgeschwindigkeit (SOS) im Punkt der minimalen Dicke des Knochens und
   - Vergleichsmittel (79) zum Vergleichen der mit Röntgenstrahlen ausgeführten Messungen mit den mit Ultraschall ausgeführten Messungen am Punkt der minimalen Dicke des Knochens.

5. Vorrichtung nach Anspruch 4, **dadurch gekennzeichnet, dass** die Röntgenstrahl-Emissionsbaugruppe (10) integral mit der Ultraschall-Strahlerbaugruppe (30) ist und die Röntgenstrahl-Empfangsbaugruppe (20) integral mit der Ultraschall-Empfangsbaugruppe (31) ist.

6. Vorrichtung nach Anspruch 4 oder 5, **dadurch gekennzeichnet, dass** die Röntgenstrahl-Emissionsbaugruppe (10) und die Ultraschall-Strahlerbaugruppe (30) auf einer ersten Auflageplatte (62) angebracht sind und die Röntgenstrahl-Empfangsbaugruppe (20) und die Ultraschall-Empfangsbaugruppe (31) auf einer zweiten Auflageplatte (62') angebracht sind, wobei die erste Auflageplatte (62) und die zweite Auflageplatte (62') verschiebbar auf Führungen (61) angebracht sind, um so in der Position einstellbar zu sein.

7. Vorrichtung nach einem der Ansprüche 4 bis 6, **dadurch gekennzeichnet, dass** die Röntgenstrahl-Emissions- (10) und die Ultraschall-Strahlerbaugruppe (30) und die Röntgenstrahl-Empfangsbaugruppe (20) und die Ultraschall-Empfangsbaugruppe (31) integral miteinander längs einer definierten Abtastbahn auf einer Ebene (X-Y) angetrieben werden.

8. Vorrichtung nach Anspruch 7, **dadurch gekennzeichnet, dass** die Röntgenstrahl-Emissions- (10) und die Ultraschall-Strahlerbaugruppe (30) und die Röntgenstrahl-Empfangs- (20) und die Ultraschall-Empfangsbaugruppe (31) auf einer Horizontalbewegungsplatte (60) angebracht sind, die in Horizontalrichtung längs einer Achse (X) verschiebbar ist, wobei die Horizontalbewegungsplatte (60) auf einer Vertikalbewegungsplatte (45) angebracht ist, die in Vertikalrichtung längs einer Achse (Y) verschiebbar ist.

9. Vorrichtung nach Anspruch 8, **dadurch gekennzeichnet, dass** die Horizontalbewegungsplatte (60) auf Schrauben (51) angebracht ist, die in Horizontalrichtung angeordnet sind und für eine Horizontalbewegung mittels eines Riemens (55) in Drehung versetzt werden, der durch einen Motor (52) angetrieben wird, und die Vertikalbewegungsplatte (45) auf Schraubenmuttern (44) angebracht ist, die mit in Vertikalrichtung angeordneten Schrauben (43) ineinander greifen, wobei die Schraubenmuttern (44) für eine Vertikalbewegung mittels eines Riemens (49) angetrieben werden, der durch einen Motor (46) angetrieben wird.

**Revendications**

1. Procédé de mesure de la densité d'un minéral osseux, comprenant les étapes suivantes :

    - balayage d'une région d'intérêt (ROI) de l'os au moyen d'un faisceau de rayons X,
    - détection des rayons X transmis dans la ROI de l'os et calcul de la densité moyenne du minéral osseux (BMD) dans ladite ROI,
    - identification d'un point d'épaisseur minimale de l'os dans la ROI,
    - balayage dudit point d'épaisseur minimale de l'os au moyen d'ultrasons,
    - calcul de l'atténuation des ultrasons (BUA) et de la vitesse du son (SOS) dans ledit point d'épaisseur minimale de l'os ; et
    - comparaison des mesures réalisées avec les rayons X aux mesures réalisées avec les ultrasons au niveau dudit point d'épaisseur minimale de l'os.

2. Procédé selon la revendication 1, **caractérisé en ce** dans que la région d'intérêt (ROI) une aire de balayage d'environ 10 cm x 10 cm est définie par les rayons X.

3. Procédé selon la revendication 1 ou 2, **caractérisé en ce qu'**il est appliqué aux os du talon.

4. Appareil (1) de mesure de la densité d'un minéral osseux, qui comprend :

    - une source de rayons X (10) destinée à balayer une région d'intérêt (ROI) de l'os au moyen d'un faisceau de rayons X,
    - un récepteur/détecteur de rayons X (20) destiné à détecter les rayons X transmis dans la ROI de l'os,
    - des moyens de calcul (79) destinés à calculer la densité moyenne d'un minéral osseux (BMD) dans ladite ROI et à identifier un point d'épaisseur minimale de l'os dans la ROI,
    - un transmetteur d'ultrasons (30) destiné à balayer ledit point d'épaisseur minimale de l'os au moyen d'ultrasons,
    - un récepteur/détecteur d'ultrasons (31) destiné à détecter les ondes ultrasonores à travers ledit point d'épaisseur minimale de l'os,
    - des moyens de calcul (79) destinés à calculer l'atténuation des ultrasons (BUA) et de la vitesse du son (SOS) dans ledit point d'épaisseur minimale de l'os, et
    - des moyens de comparaison (79) destinés à comparer les mesures réalisées avec les rayons X aux mesures réalisées avec les ultrasons au niveau dudit point d'épaisseur minimale de l'os.

5. Appareil selon la revendication 4, **caractérisé en ce que** ledit ensemble émetteur de rayons X (10) est formé en une seule pièce avec ledit ensemble transmetteur d'ultrasons (30) et ledit ensemble récepteur de rayons X (20) est formé en une seule pièce avec ledit ensemble récepteur de rayons X (31).

6. Appareil selon la revendication 4 ou 5, **caractérisé en ce que** ledit ensemble émetteur de rayons X (10) et ledit ensemble transmetteur d'ultrasons (30) sont montés sur une première plaque de support (62) et ledit ensemble récepteur de rayons X (20) et ledit ensemble récepteur d'ultrasons (31) sont montés sur une seconde plaque de support (62'), ladite première plaque de support (62) et ladite seconde plaque de support (62') étant montées avec possibilité de translation sur des guides (61) de manière à ce que la position puisse être ajustée.

7. Appareil selon l'une quelconque des revendications 4 à 6, **caractérisé en ce que** ledit émetteur de rayons X (10) et ledit ensemble transmetteur d'ultrasons (30) et ledit ensemble récepteur de rayons X (20) et ledit ensemble récepteur d'ultrasons (31) sont entraînés, dans un mouvement solidaire, le long d'une voie de balayage définie sur un plan (X-Y).

8. Appareil selon la revendication 7, **caractérisé en ce que** ledit ensemble émetteur de rayons X (10) et transmetteur d'ultrasons (30) et ledit récepteur de rayons X (20) et ledit ensemble récepteur d'ultrasons (31) sont montés sur une plaque à mouvement horizontal (60) pouvant se translater horizontalement le long d'un axe (X), ladite plaque à mouvement horizontal (60) étant montée sur une plaque à mouvement vertical (45) pouvant se translater verticalement le long d'un axe (Y).

9. Appareil selon la revendication 8, **caractérisé en ce que** ladite plaque à mouvement horizontal (60) est montée sur des vis (51) disposées horizontalement et placées en rotation au moyen d'une courroie (55) entraînée par un moteur (52) pour le mouvement horizontal et ladite plaque à mouvement vertical (45) est montée sur des vis à écrou (44) se mettant en prise dans les vis (43) disposées verticalement, lesdites vis à écrou (44) étant entraînées au moyen d'une courroie (49) entraînée par un moteur (46) pour le mouvement vertical.

FIG.2

FIG.1
PRIOR ART

FIG.4

FIG.3

EP 1 393 680 B1

FIG.5

FIG.6

FIG.7

SIGNAL ANALYSER 23

X-RAY DATA ACQUISITION 24

SIGNAL PRE AMPLIFIER 22

X-RAY DETECTOR 20

X-RAY SOURCE 10

X-RAY CONTROL 72

X-RAY POWER SUPPLY 73

ULTRASOUND DATA ACQUISITION 32

ULTRASOUND RECEIVER 31

ULTRASOUND TRANSMITTER 30

ULTRASOUND POWER SUPPLY 74

CPU 79

BUS 78

ELECTRONICS POWER SUPPLY

I/O INTERFACE 80

COMPUTER 81

PRINTER 83

KEYBOARD 84

MONITOR 82

MOUSE 85

MOTOR FOR VERTICAL MOVEMENT 46

MOTOR FOR HORIZONTAL MOVEMENT 52

VERTICAL MOVEMENT DRIVE 75

HORIZONTAL MOVEMENT DRIVE 76

DRIVE POWER SUPPLY 70

71  77

**EP 1 393 680 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 0570936 A

- US 6086538 A